# EUROPEAN PATENT SPECIFICATION

(11) **EP 0 935 460 B1**
(45) Date of publication and mention of the grant of the patent: **10.11.2004**
(21) Application number: 98916652.5
(22) Date of filing: 23.04.1998
(51) Int. Cl.: A61K 9/52, A61K 9/60

(54) **TASTE MASKED PHARMACEUTICAL COMPOSITIONS**
GESCHMACKSNEUTRALE PHARMAZEUTISCHE ZUSAMMENSETZUNGEN
COMPOSITIONS PHARMACEUTIQUES A GOUT MASQUE

(30) Priority: 23.04.1997 AU PO637197
(43) Date of publication of application: 18.08.1999
(60) Divisional application: 03078847.5
(73) Proprietor: F H Faulding & Co. Limited, Melbourne, Victoria 3000 (AU)
(72) Inventor: PITMAN, Ian, Hamilton, North Adelaide, S.A. 5006 (AU); LUKAS, Stefan, South Australia 5086 (AU); EVANS, Allen Mark, South Australia 5072 (AU); DWYER, Mark, Tasmania 7315 (AU)
(74) Representative: Setna, Rohan P.
(86) International application number: PCT/AU1998/000296
(87) International publication number: WO 1998/047493

(56) References cited:
- WO-A-88/03795
- WO-A-93/17667
- AU-A- 2 562 197
- AU-A- 4 916 185
- AU-A- 7 885 594
- AU-A- 7 950 787
- AU-A- 7 951 087
- US-A- 5 084 278
- TASIC, LJ. M. ET AL.: "The Influence of .beta.-cyclodextrin on the solubility and dissolution rate of paracetamol solid dispersions" JOURNAL OF PHARMACY & PHARMACOLOGY, vol. 44, no. 1, 1992, pages 52-55, XP002120246 ISSN 0022-3573
- PHARM. TECH. JPN., (1996), Vol. 12, No. 6, YAJIMA TOSHIO, "Particle Design Using a Spray-Dryer", pp. 873-882.
- J. MICROENCAPSULATION, (1992), Vol. 9, No. 4, FRIEND D.R., "Polyacrylate Resin Microcapsules for Taste Masking of Antibiotics", pp. 469-480.

## Description

The present invention relates to a powdered pharmaceutical formulation for the administration of pharmaceutically active compounds.

Pharmaceutical formulations for oral administration can be provided in a range of forms, including tablets, capsules, lozenges and powders. There are a number of advantages for some patients in being able to provide pharmaceuticals in powdered form and this is particularly important for patients unable to tolerate larger tablets or capsules. When a powdered form of pharmaceutical formulation is used, excipients are generally not required. Thus, a powdered formulation is particularly useful where frequent and high doses are necessary. Such as occurs in the case of analgesics.

However, the small particles which make up the powder have a large surface area and tend to release the pharmaceutical very quickly. For this reason, powders are generally not considered suitable for sustained release formulations. It is also difficult to provide powders where the pharmaceutical has an unpleasant taste since this is noticeable in the product.

Commonly, taste masking and sustained release properties are achieved in formulations by the encapsulation of the active pharmaceutical substance either in a capsule or by micro-encapsulation techniques where a polymeric coating is applied to the formulation.

The preferred method of production of powders is by way of spray drying from a solution. However, traditional teaching is that this will not produce a coated powder having sustained release properties because the coating produced is considered to be too porous. See Deasey, P.B. (1984). In: Microencapsulation and Related Drug Processes, chapter 8; pp. 181-192, Marcel Dekker, Inc. N.Y.

In US 4,767,789, ethyl cellulose has been used to coat acetaminophen to mask the bitter taste. However, the lower limit of ethylcellulose is 24% by weight and it is explicitly stated that taste masking of acetaminophen is not achieved if the ethyl cellulose falls below this limit. Spray drying processes used to coat acetaminophen fail to provide taste masking at low ethyl cellulose concentrations as the coat is generally porous and irregular with roughened surfaces and this leads to ineffective taste masking due to rapid release of the pharmaceutical from the dosage form.

Also in AU 79507/87 A ethyl cellulose has been used to coat acetaminophen by spray drying, to provide a taste-neutral powder. AU 49161/85 A discloses controlled release microparticles which may be obtained by spray drying. The microparticles comprise an active ingredient and optionally an excipient in intimate admixture with a polymer, wherein the active ingredient and the excipient are uniformly distributed throughout the matrix.

It has now been found somewhat surprisingly that powdered formulations formed by spray drying techniques can be produced satisfactorily and have suitable taste masking and sustained release properties. It is believed that the prior misconception that suitable powders could not be formed by spray drying techniques may have arisen because on the formation of tablets of the compression of the powder may have damaged the polymeric coatings.

Accordingly the present invention provides a taste-masked sustained release pharmaceutical formulation comprising spray-dried powder particles having a core element containing one or more pharmaceutically active compounds and a substantially continuous polymeric coating thereon wherein no more than 25% of the particles have a core element of less than 25 µm and no more than 2% of said particles have a core element over 250 µm and wherein said one or more pharmaceutically active compounds has a crystalline morphology with an aspect ratio of less than 3.

The present invention also provides a method of preparing pharmaceutical formulations which includes the steps of mixing a core element and a coating material in a diluent and spray drying the mixture to form a powder formulation.

Accordingly in a preferred aspect, the present invention provides a sustained release and taste masked pharmaceutical composition as described above which may provide pharmaceutic control over 24 hours.

Preferably the pharmaceutical composition includes :
approximately 90% to 77%, preferably 90 to 80% by weight, based on the total weight of the composition of a core element including at least one pharmaceutically active ingredient; and
approximately 20% to 70%, by weight of a substantially continuous coating on the core element formed from a coating material including a polymer.

The core element in the coated pharmaceutical composition according to the present invention preferably may include up to 100% by weight of the pharmaceutically active ingredient.

The core element may further include carriers or excipients, fillers, flavouring agents, stabilizing agents and/or colourants. Suitable fillers may be selected from insoluble materials such as silicon dioxide, titanium dioxide, talc, alumina, starch, kaolin, polacrilin potassium, powdered cellulose, and microcrystalline cellulose and mixtures thereof. Soluble fillers may be selected from mannitol, sucrose, lactose, dextrose, sodium chloride, sorbitol and mixtures thereof.

The filler may be present in amounts of up to approximately 75% by weight based on the total weight of the composition.

Most preferably the core element has a particle size distribution with a median of about 100µm. The particles in the distribution may vary from about 1 µm to about 250µm, more preferably from 25µm to about 250µm. Most preferably the particle size is 35 to 125µm. If the median of the distribution is close to either extreme of the distribution, the taste masking or sustained release characteristics may be affected. No more than 25% of particles will be less than 25 µm and no more than 2% will be over 250µm.

The pharmaceutically active ingredient may be selected from any one of the following:
Antacids, anti-inflammatory substances, coronary dilators, peripheral vasodilators, anti-infectives, psychotropics, anti-manics, stimulants, antihistamines, laxatives, decongestants, vitamins, gastro-intestinal sedatives, anti-diarrhoeal preparations, anti-anginal drugs, vasodilators, anti-arrhythmics, antihypertensive drugs, vasoconstrictors and migraine treatments, anti-coagulants and anti-thrombotic drugs, analgesics, anti-pyretics, hypnotics, sedatives, anti-emetics, anti-nauseates, anti-convulsants, neuromuscular drugs, hyper-and hypoglycaemic agents, thyroid and anti-thyroid preparations, diuretics, antispasmodics, uterine relaxants, mineral and nutritional additives, anti-obesity drugs, anabolic drugs, erythropoietic drugs, anti-asthmatics, bronchodilators, expectorants, cough suppressants, mucolytics, anti-ulcer and anti-uricemic drugs;
Gastro-intestinal sedatives such as metoclopramide and propantheline bromide, Antacids such as aluminium trisilicate, aluminium hydroxide and cimetidine;
Anti-inflammatory drugs such as phenylbutazone, indomethicin, naproxen, ibuprofen, flurbiprofen, diclofenac, dexamethasone, prednisone, and prednisone;
Coronary vasodilator drugs such as glyceryl trinitrate, isosorbide dinitrate and pentaerythritol tetranitrate, peripheral;
Cerebral vasodilators such as soloctidilum, vincamine, naftidrofuryl oxalate, co-dergocrine mesylate, cylandelate, papaverine and nicotine acid;
Anti-infective substances such as erythromycin stearate, cephalexin, nalidixic acid, tetracycline hydrochloride, ampicillin, flucloxacillin sodium, hexamine mandelate hexamine hippurate, and amoxacylin and vancomycin;
Neuroleptic drugs such as flurazepam, diasepam, temazepam, amitryptyline, doxepin, lithium carbonate, lithium sulfate, chlorpromazine, thioridazine, trifluperazine, fluphenazine, piperothiazine, haloperidol, maprotiline hydrochloride, imipramine and desmethylimipramine;
Central nervous stimulants such as methylphenidate, ephedrine, epinephrine, isoproterenol, amphetamine sulfate and amphetamine hydrochloride;
Antihistamic drugs such as diphenhydramine, diphenylpyraline, chlorpheniramine and brompheniramine;
Anti-diarrheal drugs such as bisacodyl and magnesium hydroxide, the laxative drug, dioctyl sodium sulfosuccinate;
Nutritional supplements such as ascorbic acid, alpha tocopherol, thiamine and pyridoxine;
anti-virals such as acyclovir;
Anti-spasmodic drugs such as dicyclomine and diphenoxylate, drugs affecting the rhythm of the heart such as verapamil, nifedipine, diltiazem, procainamide, disopyramide, bretylium tosylate, quinidine sulfate and quinidine gluconate;
Drugs used in the treatment of hypertension such as propranolol hydrochloride, guanethidine monosulphate, methyldopa, oxprenolol hydrochloride, captopril and hydralazine;
Drugs used in the treatment of migraine such as ergotamine;
Drugs affecting coagulability of blood such as epsilon aminocaproic acid and protamine sulfate;
Analgesic drugs such as acetylsalicylic acid, acetaminophen, codeine phosphate, codeine sulfate, oxycodone, dihydrocodeine tartrate, oxycodeinone, morphine, heroin, nalbuphine, butorphanol tartrate, pentazocine hydrochloride, cyclazacine, pethidine, buprenorphine, scopolamine and mefenamic acid;
Anti-epileptic drugs such as phenytoin sodium and sodium valproate;
Neuromuscular drugs such as dantrolene sodium;
Substances used in the treatment of diabetes such as tolbutamide, disbenase glucagon insulin and metformin;
Drugs used in the treatment of thyroid gland disfunction such as triiodothyronine, thyroxine and propylthiouracil;
Diuretic drugs such as furosemide, chlorthalidone, hydrochlorthiazide, spironolactone and trimterone, the uterine relaxant drug ritodrine;
Appetite supressants such as fenfluramine hydrochloride, phentermine and diethylproprion hydrochloride;
Anti-asthmatic and bronchodilator drugs such as aminophylline, theophylline, salbutamol, orciprenaline sulphate and terbutaline sulphate;
Expectorant drugs such as guaiphenesin, cough suppressants such as dextromethorphan and noscapine;
Mucolytic drugs such as carbocisteine;
Anti-septics such as cetylpyridinium chloride, tyrothricin and chlorhexidine;
Decongestant drugs such as phenylpropanolamine and pseudoephedrine, hypnotic drugs such as dichloralphenazone and nitrazepam;
Anti-nauseant drugs such as promethazine theoclate;
Haemopoietic drugs such as ferrous sulphate, folic acid and calcium gluconate; and
Uricosuric drugs such as sulphinpyrazone, allopurinol and probenecid.

Particularly preferred drugs are:

Ambroxol, ibuprofen, paracetamol, 5-amino-salicylic acid, dextromethorphan, propranolol, theophylline, diltiazem, methyldopa, pseudoephedrine, cimetidine, cephalexin, cephaclor, cephradine, naproxen, piroxicam, diazepam, diclofenac, indomethicin, amoxycillin, pivampicillin, bacampicillin, dicloxacillin, erythromycin, erythromycin stearate, lincomycin, co-dergocrine mesylate, doxycycline, dipyridamole, frusemide, triamterene, sulindac, nifedipine, atenolol, lorazepam, glibencalamide, salbutamol, trimethoprim/sulphamethoxazole, spironolactone, carbinoxamine maleate, guaiphenesin, potassium chloride and metoprolol tartrate.

Especially preferred drug includes paracetamol, cimetidine, dextromethorphan, ambroxol, risperidone, ibuprofen, amoxycillin, vancomycin, acyclovir, methyl phenidate, metformin and phenytoin.

The coating material may include a polymer including at least one of the following methyl cellulose, ethyl cellulose, hydroxypropyl cellulose, hydroxypropyl methyl cellulose, hydroxybutyl methyl cellulose, cellulose acetate, cellulose propionate (lower, medium or higher molecular weight), cellulose acetate propionate, cellulose acetate butyrate, cellulose acetate phthalate, carboxymethyl cellulose, cellulose triacetate, cellulose sulphate sodium salt, poly(methyl methacrylate), poly (ethyl methacrylate), poly (butyl methacrylate), poly (isobutyl methacrylate), poly (hexyl methacrylate), poly (phenyl methacrylate), poly (methyl acrylate), poly (isopropyl acrylate), poly (isobutyl acrylate), poly (octadecyl acrylate), poly (ethylene), poly (ethylene) low density, poly (ethylene)high density, (poly propylene), poly (ethylene glycol), poly (ethylene oxide), poly (ethylene terephthalate), poly(vinyl alcohol), poly(vinyl isobutyl ether), poly(viny acetate), poly (vinyl chloride) and polyvinyl pyrrolidone.

Preferably the polymer is a water insoluble polymer.

The water insoluble polymer preferably is selected from ethyl cellulose or dispersions of ethyl cellulose acrylic and/or methacrylic ester polymers, cellulose acetates, butyrates or propionates or copolymers of acrylates or methacrylates having a low quaternary ammonium content and the like.

Preferably the polymeric coating material includes ethyl cellulose.

The coating material according to this aspect of the present invention may further include at least one plasticiser.

The plasticiser may be selected from diethyl phthalate, triethyl citrate, triethyl acetyl citrate, triacetin, tributyl citrate, polyethylene glycol, propylene glycol, glycerol, dibutylsebacate, castor oil and the like.

The plasticiser may be present in amounts from 0 to approximately 50% by weight based on the total weight of the coating.

The coating material according to the present invention may take any suitable form which provides a continuous coating and still provides sustained release and taste masking.

The substantially continuous coat is substantially hole-free. The substantially continuous nature of the coating may be achieved by spray drying from a suspension or dispersion of the pharmaceutically active ingredient in a solution of the coating composition including a polymer in a solvent in a drying gas having a low dew point. The dew point may preferably be less than 0°C, more preferably less than approximately -15°C.

By "substantially continuous coating" we mean a coating which retains a smooth and continuous appearance when magnified 1000 times under a scanning electron microscope and wherein no holes or breakage of the coating is evident so as to reduce taste masking.

Typical coatings may be in the range of approximately 0.005 to 25 µm, preferably approximately 0.05µm to 5µm.

The solvent which may be used in the preparation of the coating of the composition may be an organic solvent. The solvent may be such that it constitutes a good solvent for the coating material but it is substantially a non-solvent or poor solvent for the pharmaceutically active ingredient. Whilst the active ingredient may partially dissolve in the solvent, in this aspect of the invention, the active ingredient will precipitate out of the solvent during the spray drying process much more rapidly than the coating material.

The solvent may be selected from alcohols such as methanol, ethanol, halogenated hydrocarbons such as dichloromethane (methylene chloride), hydrocarbons such as cyclohexane, and mixtures thereof. Dichloromethane (methylene chloride) has been found to be particularly suitable.

The concentration of polymer in the solvent will normally be less than 75% by weight. Normally the concentration will be in the range of 10-30% by weight.

Where the polymer is ethyl cellulose, the solvent is preferably methylene chloride. The concentration of ethyl cellulose is preferably in the range of 5-10% most preferably 7% by weight based on the total concentration of the coating material.

The pharmaceutically active ingredient, provided in a form suitable for coating may be suspended in the coating material/organic solvent solution, preferably in an ethyl cellulose/methylene chloride solution at a concentration in the range of 10-30% by weight, preferably in the range of 14-20% by weight.

Shape can influence the coverage and stability of the coat. Sharp angles on a crystal can cause weaknesses in the coat. These sharp corners may lead to stress points on the coat and cause weaknesses in the structure possibly leading to premature release of the pharmaceutical from the pharmaceutical composition.

Where the coat is thinner at the vertices this leads to more rapid release.

The composition according to the present invention is applicable to pharmaceutically active ingredients having a crystalline morphology and particularly a low aspect ratio. The aspect ratio is a measure of the length compared to the breadth. For example, an aspect ratio of 1 would be a box or sphere. The higher the aspect ratio, the more pointy and needle-like crystals will be.

The crystal geometry may result in a relatively thin coat at the crystal needle tips the release rates may be more rapid than is preferred with such actives. Similarly, where the pharmaceutically active ingredient exhibits high water or organic solvent solubility, the release rates may be more rapid than is required in a particular application. Furthermore, areas of thin coating are susceptible to breaking and cracking and hence ineffective for sustained release and taste masking.

Applicants have found that a spherical shape of the particle is most advantageous for both stability of the coat and high payload of active pharmaceutical. Therefore, the aspect ratio is less than 3, more preferably 1 to 2 and most preferably approximately 1 providing a substantially rounded shape. More preferably, the aspect ratio is 1 and the shape is round.

It is also preferable for all particles to be of the same size and shape. Inconsistencies in size and shape can lead to inconsistent coating. Where the drug particles are of different size and shape, polymeric coating materials such as ethyl cellulose will deposit differently on each particle. It is therefore preferable to have all particles the same size and shape so that the coating process is better controlled and maintained.

Accordingly, in a preferred form, the composition may include a core element comprising approximately 30% to 80% by weight based on the total weight of the composition, said core element including:
approximately 52 to 85% by weight of a pharmaceutically active ingredient; and
approximately 5% to 25% by weight of a supplementary component selected from waxes, water insoluble polymers, enteric polymers, and partially water soluble polymers and other suitable pharmaceutical excipients.

The supplementary component may be provided as an intimate mixture with the active ingredient or as a precoat thereon. Where an intimate mixture is formed, polymers such as hydroxypropyl methyl cellulose may be used.

Where a precoat is formed, a wax coat is preferred. A paraffin wax or a canauba wax may be used. In a preferred form the pharmaceutically active ingredient is a compound of high water or solvent solubility and the supplementary component forms a precoat on the active ingredient.

Spray drying of the pharmaceutically active ingredient and polymer in the solvent involves spraying a stream of air into an atomised suspension so that solvent is caused to evaporate leaving the pharmaceutical drug coated with the polymer coating material.

Preferably, for a solvent such as methylene chloride, the solvent concentration in the drying chamber is maintained above 40,000 parts, more preferably in the range of approximately 40,000 to 100,000 parts per million of organic solvent.

The spray-drying process for such solvents may be conducted at a process temperature of from approximately 5°C to 35°C.

The utilisation of a drying gas exhibiting a low dew point aids the production of a substantially continuous coating. It has also been found that the presence of a solvent during the drying step slows the evaporation rate of the solvent such that a substantially continuous coat exhibiting reduced permeability is produced. The concentration of non-solvent (e.g. water) present should be kept very low and that, in combination with the controlled drying conditions, results in microcapsules with continuous coats. These two factors may be interrelated. Thus the higher the drying gas dew point, the higher the solvent vapour pressure required in the system to give a substantially continuous coat.

The drying process may be of any suitable type.

Spray drying of the pharmaceutical compositions may be undertaken utilising either rotary, pneumatic or pressure atomisers located in either a cocurrent, counter-current or mixed-flow spray dryer or variations thereof.

The drying gas may be heated or cooled to control the rate of drying. A temperature below the boiling point of the solvent may be used. Inlet temperatures will typically be in the range of from approximately 40°C to 120°C and outlet temperatures approximately 5°C to 35°C.

The present invention permits the optimisation of the coat formation to meet the needs of the material or application. Adjusting the coating composition allows modification of the release profile for the material. Controlling the process parameters including temperature, solvent concentration, spray dryer capacity, atomising air pressure, droplet size, viscosity, total air pressure in the system and solvent system, allows the formation of a range of coats, ranging from dense, continuous, non-porous coats through to more porous microcapsule/polymer matrices.

The spray drying process may utilise a method employing a nozzle to atomise the drugs in polymeric coating material/organic solvent solution. Preferably pneumatic atomisation is used. The nozzle produces individual droplets with a single unit of drug suspended in a polymeric coating material/organic solvent solution. Removal of the organic solvent results in a drug dosage unit coated with the polymeric coating material.

Preferably the nozzle is a 2 fluid nozzle. The ratio of solvent/drug to air is important in a 2 fluid nozzle and this may be varied by optimizing the relative positions of the outlet and inner passages. The operating conditions include variations on air inlet temperatures, air outlet temperatures, air pressures, feed rates of solvent and drug suspensions, atomisation, air quality and outlet diameters of inlet and outlet passages of the atomizer. Preferably, the air inlet temperature is approx 70-150°C, the air outlet temperature is in the range of 20-50°C, the air flow rate is in the range of 40-1300kg/hr, the feed rates of solvent and drug is in the range of 3-75 kg/hr, atomisation air quantity is in the range of 6-60 kg/hr and the outlet diameter of the inlet and outlet passages are approximately 2-6 mm and 4-12 mm in diameter respectively.

More preferably, the air inlet temperature is approx 100°C, the air outlet temperature is in the range of 25-35°C, the air flow rate is in the range of 40-80kg/hr, the feed rates of solvent and drug is in the range of 8-9 kg/hr, atomisation air quantity is in the range of 7-9 kg/hr and the outlet diameter of the inlet and outlet passages are approximately 2-3 mm and 4-6 mm in diameter respectively.

The product may be collected by any means available to the skilled addressee. Preferably the collection method is by sock filters or cyclone collection.

Accordingly, the present invention further provides in a preferred aspect a post-treatment step to remove residual solvent. The post treatment may include a post drying step including drying the final product on a tray and drying the product at a bed temperature sufficient to remove excess solvent but not degrade the pharmaceutical drug. Preferably the temperature is in the range of 35°C to 45°C, most preferably at 40°C.

The pharmaceutical composition may be in the form of a powder with a particle size distribution in the range of 0.1µm to 250µm, most preferably in the range of 35µm to 125µm. The small particle size ensures that the particles have a substantially non-gritty feel in the mouth. The small particle size may also minimise break-up of the particles in the mouth, eg by the teeth. When in the form of a powder, the pharmaceutical composition may be administered directly into the mouth or mixed with a carrier such as water, or semi-liquid compositions such as syrups, yoghurt. Preferably, the pharmaceutical composition is a powder which is mixed with water prior to ingestion.

The taste masked pharmaceutical composition may be further provided in any suitable unit dosage form.

Because of the sustained release characteristics of the pharmaceutical composition, it can be used as a means to treat disorders in which relief is required over a period of time. Examples of disorders include bacterial infections; pain-related disorders including arthritis, rheumatism, muscle pain; viral infections; depressants; diabetes and epilepsy. Pharmaceuticals useful in treating these disorders include antibiotics such as amoxycillin or vancomycin; analgesics such as paracetamol or ibuprofen; antivirals such as acyclovir; stimulants such as methylphenidate; antidiabetics such as metformin and antiepileptics such as phenytoin.

The present invention will now be more fully described with reference to the accompanying examples.

### Example 1

### Paracetamol Formulation - Nopap Powder

Ethyl cellulose was dissolved in methylene chloride and then paracetamol dispersed in the solution, in the following formulation, to produce a slurry.

| | |
|---|---|
| Ethyl cellulose N10 NF | 7% w/w |
| Paracetamol | 28% w/w |
| Methylene Chloride | 65% w/w |

This slurry is then spray dried under the following process conditions in a NIRO "PM" type 2 fluid atomiser.

| | |
|---|---|
| Fluid Insert | 1.3mm |
| Air Cap | 5.0mm |
| Feed Rate | 3.0 kg/hr |
| Atomising gas flow rate | 5 - 6 m³/hr |
| Process gas Inlet Temperature | 40°C |
| Process gas flow rate | 20 m³/hr |

The final formulated product is a white, free flowing taste masked powder consisting of 80% paracetamol and 20% ethyl cellulose with a median particle size of less than 150µm.

### Example 2

### Pharmacokinetic Parameters from a single 1000mg dose of Tylenol Extra Strength Tablet vs Test Coated Paracetamol Powder (Nopap Powder)

A pilot study of 6 healthy males was conducted to evaluate pharmacokinetic parameters following injestion of 1000mg of a single dose of Tylenol Extra Strength Tablet (immediate release) and Test Coated Paracetamol Powder (Nopap) (sustained release, prepared according to Example 1).

### METHODS

1000 mg of Tylenol® Extra Strength Tablet or Test Coated Paracetamol (Nopap) prepared according to Example 1 were administered to 6 healthy males. Plasma paracetamol concentrations were measured under fasted and fed conditions.

Tables 1, 2 and 3 summarise statistical comparisons. The arithmetic mean and individual pharmacokinetic parameters for each study treatment are shown in Table 4.

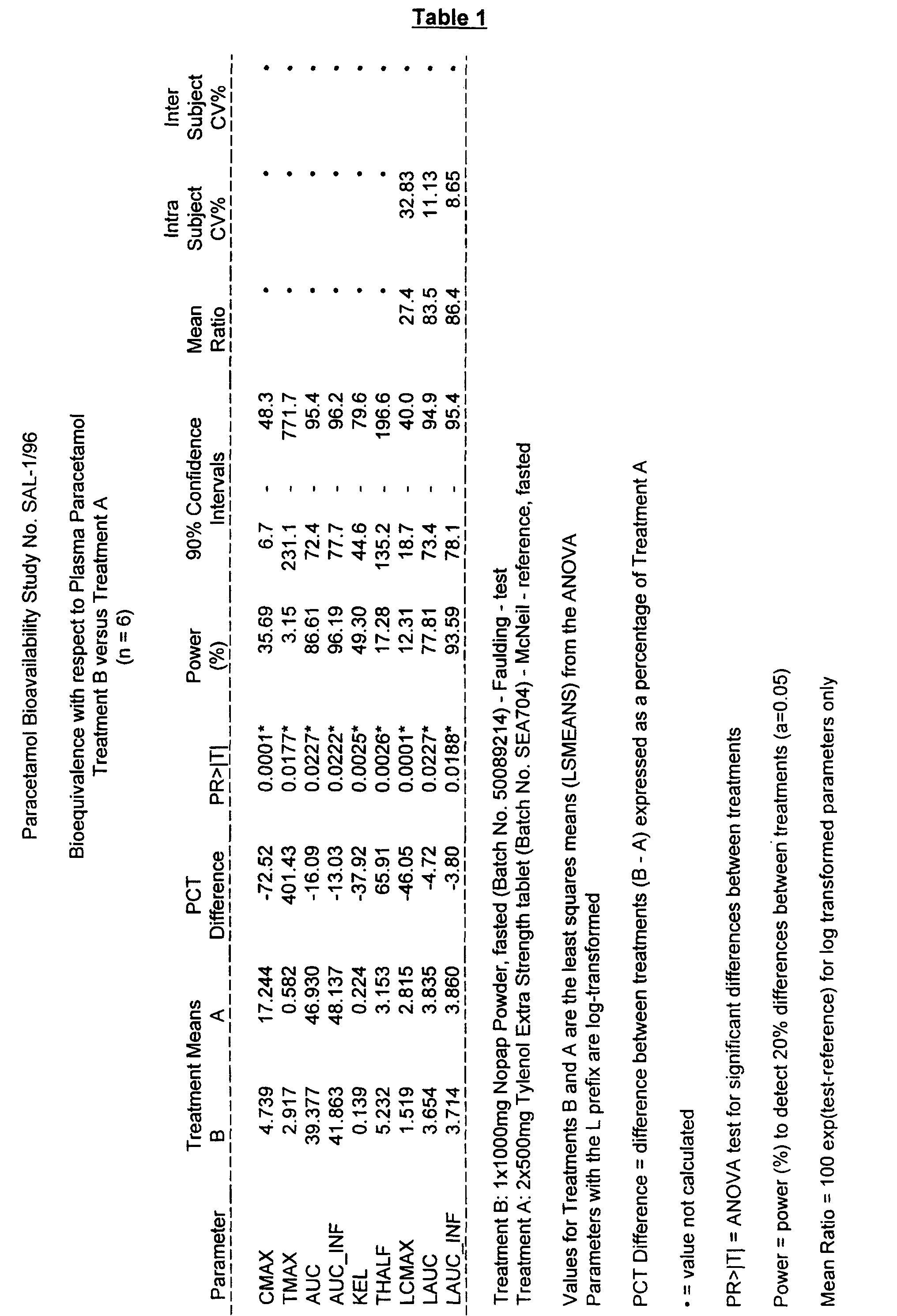

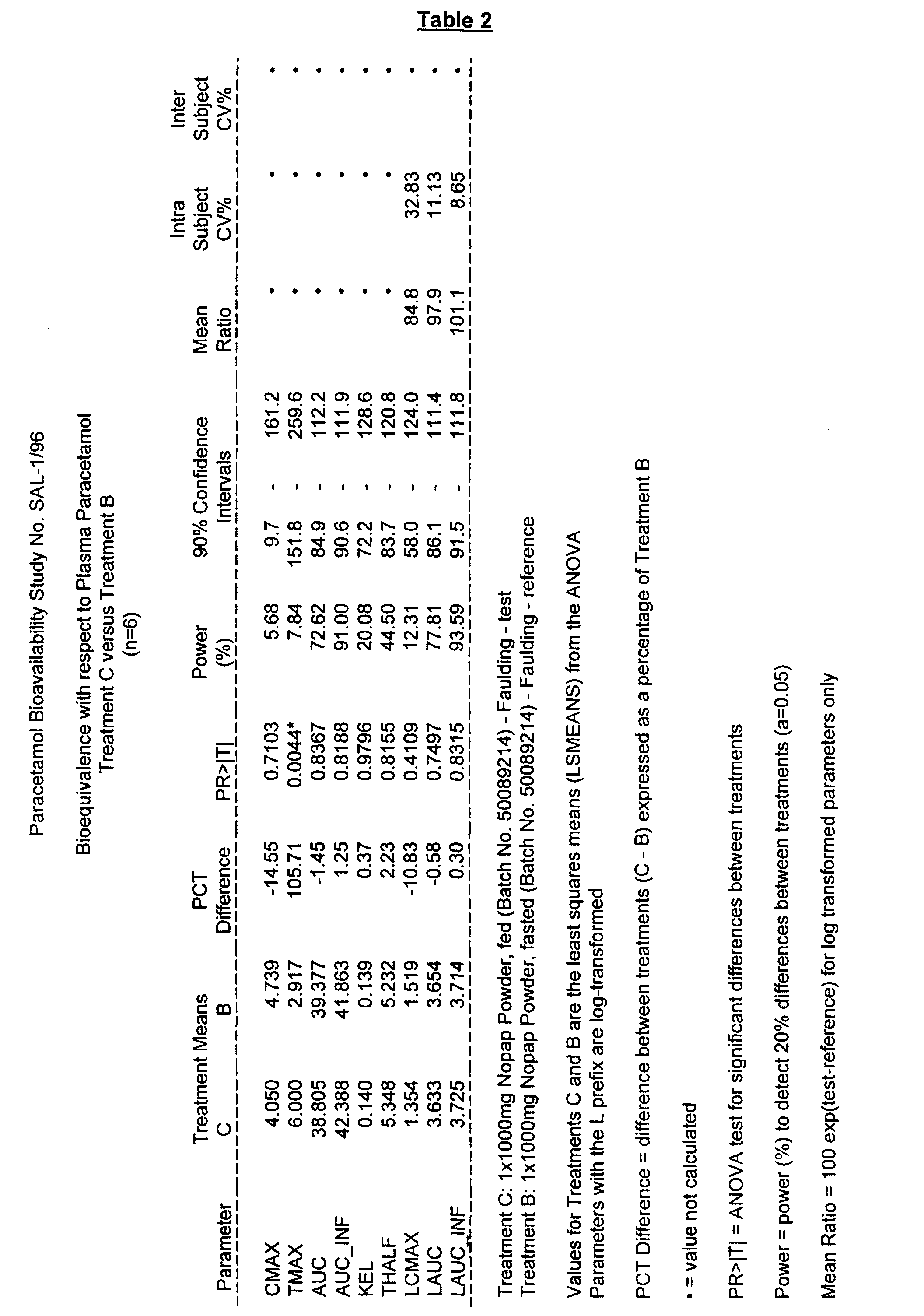

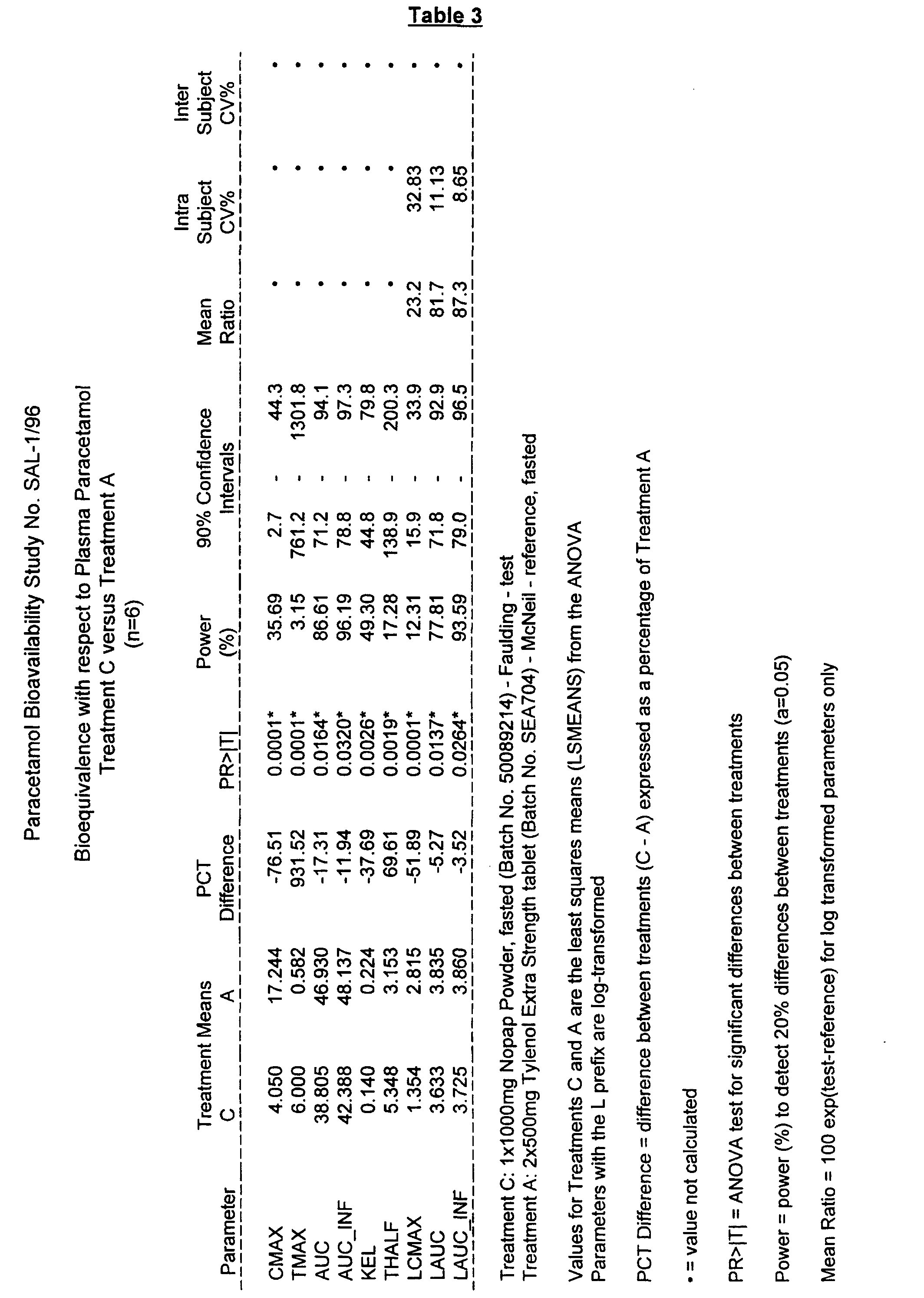

**Table 4**

| Study Design: Single dose (1000 mg) in 6 healthy volunteers with blood sampling over 24 hours. | | | | |
|---|---|---|---|---|
| TREATMENT A: Tylenol (fasted) | | | | |
| Subject | CMAX(mg/L) | TMAX(hours) | AUC(mg.h/L) | AUC-INF(mg.h/L) |
| 1 | 14.032 | 0.67 | 51.18 | 51.88 |
| 2 | 17.996 | 0.33 | 53.94 | 55.62 |
| 3 | 10.011 | 1.5 | 34.29 | 34.96 |
| 4 | 19.322 | 0.33 | 49.27 | 50.93 |
| 5 | 20.513 | 0.33 | 39.9 | 40.97 |
| 6 | 21.592 | 0.33 | 53 | 54.56 |
| MEAN | 17.244 | 0.58 | 46.93 | 48.14 |

| TREATMENT B: Nopap Powder (fasted) | | | | |
|---|---|---|---|---|
| Subject | CMAX(mg/L) | TMAX(hours) | AUC(mg.h/L) | AUC-INF(mg.h/L) |
| 1 | 5.235 | 4 | 45.71 | 48.04 |
| 2 | 4.081 | 3 | 42.81 | 46.75 |
| 3 | 7.332 | 1.5 | 30.41 | 31.82 |
| 4 | 4.815 | 3.5 | 49.75 | 53.46 |
| 5 | 3.122 | 2.5 | 29.76 | 31.1 |
| 6 | 3.85 | 3 | 37.82 | 40.01 |
| MEAN | 4.739 | 2.92 | 39.38 | 41.86 |

| TREATMENT C: Nopap Powder (fed) | | | | |
|---|---|---|---|---|
| Subject | CMAX(mg/L) | TMAX(hours) | AUC(mg.h/L) | AUC-INF(mg.h/L) |
| 1 | 3.127 | 11 | 35.79 | 43.46 |
| 2 | 3.637 | 6 | 45.06 | 48.88 |
| 3 | 2.824 | 5 | 30.51 | 33.64 |
| 4 | 6.846 | 4 | 53.27 | 55.14 |
| 5 | 4.239 | 5 | 27.5 | 29.44 |
| 6 | 3.626 | 5 | 40.7 | 43.77 |
| MEAN | 4.050 | 6.00 | 38.81 | 42.39 |

### DISCUSSION OF RESULTS

In evaluating formulations to determine bioequivalence, the 90% confidence intervals and mean ratios of the In-transformed pharmacokinetic parameters CMAX, AUC and AUC-INF are compared.

### (a) Comparison of Reference Tylenol Extra Strength Tablet (Fasted) vs Test Nopap Powder (Fasted) - refer Table 1

The 90% confidence interval and mean ratio for CMAX fell outside the allowed bioequivalence range of 80-125% and the difference was statistically significant, as would be expected for a sustained-release formulation compared with an immediate-release formulation. In fact, the mean CMAX value showed approximately a 70% reduction. Although the 90% confidence intervals for In-transformed AUC and AUC-INF fell outside the lower limit allowed for bioequivalence and the difference was statistically significant for both parameters, the mean ratio values, which are a measure of bioavailability, were within the 80-125% "bioequivalence" range for both "extent of absorption" parameters (83.5% and 86.4% for AUC and AUC-INF, respectively). The mean TMAX values were 2.92 hours for Nopap powder and 0.58 hours for Tylenol Tablet and the difference was statistically significant, as would be expected of a sustained-release formulation compared with an immediate-release formulation.

Thus, under fasted conditions, Nopap powder exhibits sustained-release characteristics compared with Tylenol tablets with a significantly reduced rate of paracetamol absorption as evidenced by a significant reduction in CMAX and significant increase in TMAX. Only one subject, showed a reduced CMAX with Nopap powder (fasted) compared with Tylenol Extra Strength tablet (fasted), without an increase in TMAX (1.50 hours for both formulations).

### (b) Comparison of Test Nopap Powder (Fasted) vs (Fed) - refer Table 2

The 90% confidence interval for CMAX fell outside the allowed bioequivalence range of 80-125%, however, the mean ratio value (84.8%) was included in the allowed bioequivalence range. In addition, food did not cause a significant reduction in CMAX (p>0.05). The 90% confidence intervals for In-transformed AUC and AUC-INF fell within the range allowed for bioequivalence, the differences were not statistically significant, and the mean ratio values, which are a measure of bioavailability, were within the 80-125% "bioequivalence" range for both "extent of absorption" parameters (97.9% and 101.1% for AUC and AUC-INF, respectively). The mean TMAX values of 6.00 hours for Nopap powder (fed) and 2.92 hours for Nopap powder (fasted) were statistically significantly different.

In accordance with FDA 1992 Bioequivalence Guidelines, for a sustained-release product to demonstrate a comparable food effect, the mean ratios of the In-transformed least squares mean pharmacokinetic parameters AUC, AUC-INF and CMAX must fall within the 80-125% range. Therefore, based on these guidelines, Nopap powder is bioequivalent when administered under fasted and fed conditions, with the only effect of food being a significant lengthening of TMAX.

Table 3, summarises the comparison of Tylenol Extra Strength Tablet (Fasted) vs Tested Nopap Powder (Fed).

### Example 3

### Paracetamol Powder - Steady State Simulations

A single dose study based on results of Example 2 were used to predict 24 hour plasma concentration. Plasma concentration versus time profiles for twice daily administration of coated paracetamol powder (according to Example 1) were analysed.

In simulated studies coated paracetamol powder was administered as a dose of 2g every 12 hours. Hence, the total daily dose (4g) is in keeping with current dose recommendations for paracetamol in adults.

When dosed at a level of 2g twice a day, the plasma concentrations of paracetamol do not fall below 4mg/L and remain well below 20mg/L. As noted in a review by Prescott [Paracetamol, A Critical Bibliographic review, Taylor & Francis, London, 1996, page 228-229] the therapeutic range for effective analgesia is about 5 to 20mg/L, with a similar range for antipyretic activity.

Furthermore, during repeated administration of conventional paracetamol at a dose of 1g every 6 hours (4g a day in 4 divided doses) the mean trough plasma concentration (immediately pre-dose) was 3 mg/L, and the mean maximum concentration was about 12 mg/L [Nielson *et al*. (1991) British Journal of Clinical Pharmacology 31: 267-270]. Accordingly in the coated paracetamol the predicted steady-state levels for coated paracetamol powder are within this range.

In conclusion, the preliminary results suggest that the plasma concentrations of paracetamol obtained during twice daily administration of coated paracetamol powder will be within the range of concentrations encountered with four times daily dosing with conventional paracetamol formulations. Twice daily dosing with coated paracetamol powder according to the present invention would provide good antipyretic and analgesic control over 24 hours. Perhaps the most important advantage is overnight pain relief, particularly for patients with arthritic conditions leading to morning stiffness.

### Example 4

A slurry was produced with the following composition:

| | |
|---|---|
| Clarithromycin | 50 gm |
| Eudragit RS100 | 50 gm |
| Ethanol | 500 gm |
| Sodium Lauryl Sulphate | 2 gm |

The slurry was spray dried at an gas inlet temperature of 100°C to produce a free flowing fine powder which had satisfactory sustained release properties and adequate taste masking of the clarithromycin.

## Claims

1. A taste-masked sustained release pharmaceutical formulation comprising spray-dried powder particles having a core element containing one or more pharmaceutically active compounds and a substantially continuous polymeric coating thereon wherein no more than 25% of the particles have a core element of less than 25 µm and no more than 2% of said particles have a core element over 250 µm and wherein said one or more pharmaceutically active compounds has a crystalline morphology with an aspect ratio of less than 3.

2. The pharmaceutical formulation of claim 1 wherein said aspect ratio is from 1 to 2.

3. The pharmaceutical formulation of claim 1 or claim 2 wherein said aspect ratio is approximately 1 providing a substantially rounded shape.

4. The pharmaceutical formulation of any preceding claim wherein said aspect ratio is 1 and the shape is round.

5. The pharmaceutical formulation of any preceding claim wherein said core element has a particle size between 0.1 µm and 250 µm.

6. The pharmaceutical formulation of claim 5 wherein said particle size is 35 to 125 µm.

7. The pharmaceutical formulation of any preceding claim wherein said coating comprises less than 20% of the weight of the formulation.

8. The formulation according to any preceding claim wherein said polymer coating is an ethyl cellulose coating.

9. The pharmaceutical formulation of any preceding claim wherein the thickness of said coating is within the range of from 0.005 to 25 µm.

10. The pharmaceutical formulation of any preceding claim wherein said pharmaceutically active compound is paracetamol.

11. The pharmaceutical formulation of any one of claims 1 to 9 wherein said pharmaceutically active compound is clarithromycin.

12. A method of preparing a pharmaceutical formulation of any preceding claim comprising the steps of mixing said core element and said coating in a diluent and spray-drying said mixture to form a powder.

## Patentansprüche

1. Geschmacksneutrale pharmazeutische Zusammensetzung mit verzögerter Freisetzung, umfassend sprühgetrocknete Pulverteilchen mit einem Kernelement, das eine oder mehrere pharmazeutisch aktive Verbindungen enthält, und einen im Wesentlichen durchgängigen polymeren Überzug, wobei nicht mehr als 25% der Teilchen ein Kernelement von weniger als 25 µm und nicht mehr als 2% der Teilchen ein Kernelement von mehr als 250 µm aufweisen, und wobei die eine oder mehrere pharmazeutisch aktive(n) Verbindung(en) eine Kristallmorphologie mit einem Aspektverhältnis von weniger als 3 haben.

2. Pharmazeutische Zusammensetzung nach Anspruch 1, worin das Aspektverhältnis zwischen 1 und 2 beträgt.

3. Pharmazeutische Zusammensetzung nach Anspruch 1 oder 2, worin das Aspektverhältnis etwa 1 beträgt und sich eine im Wesentlichen runde Form ergibt.

4. Pharmazeutische Zusammensetzung nach einem der vorhergehenden Ansprüche, worin das Aspektverhältnis 1 und die Form rund ist.

5. Pharmazeutische Zusammensetzung nach einem der vorhergehenden Ansprüche, worin das Kernelement eine Teilchengröße zwischen 0,1 µm und 250 µm aufweist.

6. Pharmazeutische Zusammensetzung nach Anspruch 5, worin die Teilchengröße 35 bis 125 µm ist.

7. Pharmazeutische Zusammensetzung nach einem der vorhergehenden Ansprüche, worin der Überzug weniger als 20 Gew.-% des Gewichts der Zusammensetzung entspricht.

8. Zusammensetzung nach einem der vorhergehenden Ansprüche, worin der polymere Überzug ein Ethylcelluloseüberzug ist.

9. Pharmazeutische Zusammensetzung nach einem der vorhergehenden Ansprüche, worin die Dicke des Überzugs im Bereich von 0,005 bis 25 µm liegt.

10. Pharmazeutische Zusammensetzung nach einem der vorhergehenden Ansprüche, worin die pharmazeutisch aktive Verbindung Paracetamol ist.

11. Pharmazeutische Zusammensetzung nach einem der Ansprüche 1 bis 9, worin die pharmazeutisch aktive Verbindung Clarithromycin ist.

12. Verfahren zur Herstellung einer pharmazeutischen Zusammensetzung nach einem der vorhergehenden Ansprüche, umfassend die Schritte des Mischens des Kernelements und des Überzugs in einem Verdünnungsmittel und Sprühtrocknen des Gemisches, um ein Pulver zu erzeugen.

## Revendications

1. Composition pharmaceutique à libération prolongée à goût masqué comprenant des particules de poudre séchées par pulvérisation ayant un noyau contenant un ou plusieurs composés actifs et un enrobage polymère essentiellement continu sur celui-ci, dans laquelle pas plus de 25 % des particules ont un noyau inférieur à 25 µm et pas plus de 2 % desdites particules ont un noyau de plus de 250 µm, ledit ou lesdits composés pharmaceutiquement actifs ayant une morphologie cristalline avec un rapport dimensionnel inférieur à 3.

2. Composition pharmaceutique selon la revendication 1, dans laquelle ledit rapport dimensionnel est de 1 à 2.

3. Composition pharmaceutique selon la revendication 1 ou la revendication 2, dans laquelle ledit rapport dimensionnel est d'environ 1, conférant une forme essentiellement arrondie.

4. Composition pharmaceutique selon l'une quelconque des revendications précédentes, dans laquelle ledit rapport dimensionnel est de 1 et la forme est arrondie.

5. Composition pharmaceutique selon l'une quelconque des revendications précédentes, dans laquelle ledit noyau a une taille de particule comprise entre 0,1 µm et 250 µm.

6. Composition pharmaceutique selon la revendication 5, dans laquelle ladite taille de particule est de 35 à 125 µm.

7. Composition pharmaceutique selon l'une quelconque des revendications précédentes, dans laquelle ledit enrobage comprend moins de 20 % en poids de la composition.

8. Composition pharmaceutique selon l'une quelconque des revendications précédentes, dans laquelle ledit enrobage polymère est un enrobage d'éthylcellulose.

9. Composition pharmaceutique selon l'une quelconque des revendications précédentes, dans laquelle l'épaisseur dudit enrobage est de l'ordre de 0,005 à 25 µm.

10. Composition pharmaceutique selon l'une quelconque des revendications précédentes, dans laquelle ledit composé pharmaceutiquement actif est le paracétamol.

11. Composition pharmaceutique selon l'une quelconque des revendications 1 à 9, dans laquelle ledit composé pharmaceutiquement actif est la clarithromycine.

12. Procédé de préparation d'une composition pharmaceutique selon l'une quelconque des revendications précédentes, comprenant les étapes du mélange dudit noyau et dudit enrobage dans un diluant et le séchage par pulvérisation dudit mélange pour former une poudre.
